# EUROPÄISCHE PATENTSCHRIFT

(11) **EP 4 393 900 B1**
(45) Veröffentlichungstag und Bekanntmachung des Hinweises auf die Patenterteilung: **28.05.2025**
(21) Anmeldenummer: 22216759.5
(22) Anmeldetag: 27.12.2022
(51) Int. Cl.: C07B 41/06, C07C 45/50, C07C 47/02, C07F 9/6574, B01J 31/18

(54) **DIPHOSPHITE MIT CIS-STÄNDIGEN SUBSTITUENTEN**
DIPHOSPHITES WITH CIS-POSITION SUBSTITUENTS
DIPHOSPHITES PRESENTANT DES SUBSTITUANTS CIS-TERMINAUX

(43) Veröffentlichungstag der Anmeldung: 03.07.2024
(73) Patentinhaber: Evonik Oxeno GmbH & Co. KG, 45772 Marl (DE)
(72) Erfinder: SALE, Anna Chiara, 45657 Recklinghausen (DE); FRANKE, Robert, 45772 Marl (DE); FRIDAG, Dirk, 45721 Haltern am See (DE); KUCMIERCZYK, Peter, 44628 Herne (DE); MARKOVIC, Ana, 45721 Haltern am See (DE); BÖRNER, Armin, 18059 Rostock (DE); HOLZ, Jens, 18196 Kessin (DE)
(74) Vertreter: Evonik Patent Association

(56) Entgegenhaltungen:
- WO-A1-2008/071508

## Beschreibung

Die Erfindung betrifft Diphosphite mit cis-ständigen Substituenten und deren Verwendung in der Hydroformylierung.

Phosphorhaltige Verbindungen spielen als Liganden in einer Vielzahl von Reaktionen eine entscheidende Rolle, z.B. in der Hydrierung, in der Hydrocyanierung und auch in der Hydroformylierung.

Die Reaktionen zwischen Olefinverbindungen, Kohlenmonoxid und Wasserstoff in Gegenwart eines Katalysators zu den um ein C-Atom reicheren Aldehyden ist als Hydroformylierung bzw. Oxierung bekannt. Als Katalysatoren in diesen Reaktionen werden häufig Verbindungen der Übergangsmetalle der VIII. Gruppe des Periodensystems der Elemente verwendet. Bekannte Liganden sind beispielsweise Verbindungen aus den Klassen der Phosphine, Phosphite und Phosphonite mit jeweils dreiwertigem Phosphor P^{III}. Eine gute Übersicht über den Stand der Hydroformylierung von Olefinen findet sich in R. Franke, D. Selent, A. Börner, "Applied Hydroformylation", Chem. Rev., 2012 (112), 11, 5675-5732, DOI:10.1021/cr3001803. WO 2008/071508 A1 offenbart Verbindungen, die in Hydroformylierungsreaktionen verwendet kann.

Die technische Aufgabe der Erfindung ist die Bereitstellung einer Verbindung, mit welcher bei der Hydroformylierung von Olefinen eine gesteigerte n-Selektivität erzielt werden kann. Das bedeutet, dass das Verhältnis von n-Produkt zu iso-Produkt gesteigert werden soll.

Die Aufgabe wird gelöst durch eine Verbindung gemäß Anspruch 1.

Verbindung gemäß Formel (I): 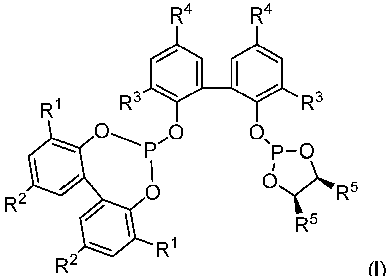
wobei R¹, R², R³, R⁴ ausgewählt sind aus: -(C₁-C₁₂)-Alkyl, -O-(C₁-C₁₂)-Alkyl und
R⁵ ausgewählt ist aus: -(C₁-C₁₂)-Alkyl, -O-(C₁-C₁₂)-Alkyl, -Ph.

In einer Ausführungsform ist R¹ ausgewählt aus: -CH₃, -OCH₃, -*^{tert}*Bu.

In einer Ausführungsform steht R¹ für -*^{tert}*Bu.

In einer Ausführungsform ist R² ausgewählt aus: -CH₃, -OCH₃, -*^{tert}*Bu.

In einer Ausführungsform steht R² für -*^{tert}*Bu.

In einer Ausführungsform ist R³ ausgewählt aus: -CH₃, -OCH₃, -*^{tert}*Bu.

In einer Ausführungsform steht R³ für -*^{tert}*Bu.

In einer Ausführungsform ist R⁴ ausgewählt aus: -CH₃, -OCH₃, -*^{tert}*Bu.

In einer Ausführungsform steht R⁴ für -*^{tert}*Bu.

In einer Ausführungsform ist R⁵ ausgewählt aus: -CH₃, -OCH₃, -^{*ter*t}Bu, -Ph.

In einer Ausführungsform steht R⁵ für -Ph.

In einer Ausführungsform weist die Verbindung die Struktur (1) auf:

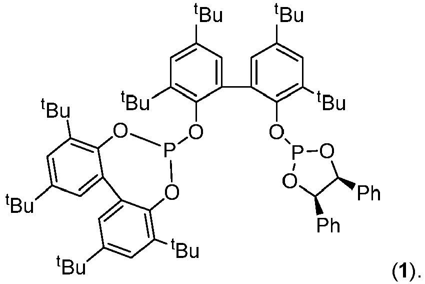

Neben der Verbindung selbst, wird auch ein Verfahren beansprucht, in welchen die zuvor beschriebenen Verbindungen zum Einsatz kommen.

Verfahren umfassend die Verfahrensschritte:
a) Vorlegen eines Olefins;
b) Zugabe einer zuvor beschriebenen Verbindung;
c) Zugabe einer Rh-Verbindung;
d) Zuführen von H₂ und CO;
e) Erwärmen des Reaktionsgemisches aus a) bis d), wobei das Olefin zu einem Aldehyd umgesetzt wird.

In einer Variante des Verfahrens ist die Rh-Verbindung ausgewählt aus: Rh(acac)(CO)₂, [(acac)Rh(COD)] (Umicore, acac = Acetylacetonat-Anion; COD = 1,5-Cyclooctadien), Rh₄CO₁₂.

In einer Variante des Verfahrens ist die Rh-Verbindung Rh(acac)(COD).

In einer Variante des Verfahrens erfolgt das Zuführen von H₂ und CO in Verfahrensschritt d) mit einem Druck in dem Bereich von 1 bis 6 MPa (10 bis 60 bar).

In einer Variante des Verfahrens erfolgt das Zuführen von H₂ und CO in Verfahrensschritt d) mit einem Druck in dem Bereich von 1,5 bis 4,5 MPa (15 bis 45 bar).

In einer Variante des Verfahrens erfolgt das Erwärmen des Reaktionsgemisches in Verfahrensschritt e) auf eine Temperatur in dem Bereich von 80 °C bis 160 °C.

In einer Variante des Verfahrens erfolgt das Erwärmen des Reaktionsgemisches in Verfahrensschritt e) auf eine Temperatur in dem Bereich von 100 °C bis 140 °C.

Im Folgenden soll die Erfindung anhand eines Ausführungsbeispieles näher erläutert werden.

### Synthese (1): 2,4,8,10-Tetra-tert.-butyl-6-((3,3',5,5'-tetra-tert.-butyl-2'-(((4S,5R)-4,5-diphenyl-1,3,2-dioxaphospholan-2-yl)oxy)-[1,1'-bipheny1]-2-yl)oxy)dibenzo[d,f][1,3,2]dioxaphosphepin

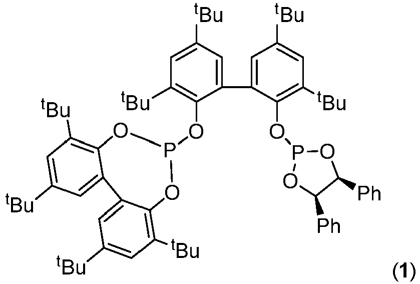

Zu einer Lösung von 3,3',5,5'-Tetra-*tert*.-butyl-2'-((2,4,8,10-tetra-*tert*.-butyldibenzo [*d,f*][1,3,2]dioxaphosphepin-6-yl)oxy)-[1,1'-biphenyl]-2-ol (0.430 g, 0.506 mmol) in THF (3 ml) wurden bei -20 °C eine Lösung von n-BuLi (0.532 M Lösung in Hexan, 1.00 ml, 0.532 mmol) getropft. Nach 20 min ließ man die Reaktionslösung auf 0 °C erwärmen und versetzte diese anschließend tropfenweise mit einer Lösung von (4R,5S)-2-chloro-4,5-diphenyl-1,3,2-dioxaphospholane (0.148 g, 0.532 mmol) in THF (2 ml). Nach dem Rühren über Nacht bei Raumtemperatur wurde das Lösemittel unter Vakuum entfernt und der weiße Rückstand mit Toluol (6 ml) versetzt und die ungelösten Bestandteile abfiltriert. Das Filtrat wurde erneut unter Vakuum eingeengt und das Rohprodukt bei 60 °C getrocknet. Zur Reinigung wurde dieser in Acetonitril (5 ml) in der Siedehitze gelöst, nach Abkühlen der ausgefallene Niederschlag abfiltriert und das Produkt als weißer Feststoff isoliert (0.22 g, 0.202 mmol, 40% Ausbeute).

Elementaranalyse (berechnet für C₇₀H₉₂O₆P₂ = 1091.448 g/mol): C = 77.22 % (77.03 %); H = 8.45 % (8.50 %); P = 5.68 % (5.68 %).
ESI-TOF HRMS: m/z= 1090.6369
[M⁺+H], berechnet m/z= 1091.6448 (gefunden 1091.6442)
[M⁺+Na], berechnet m/z= 1113.6262 (gefunden 1113.6256)

### Synthese (2): 2,4,8,10-Tetra-tert.-butyl-6-((3,3',5,5'-tetra-tert.-butyl-2'-(((4R,5R)-4,5-diphenyl-1,3,2-dioxaphospholan-2-yl)oxy)-[1,1'-biphenyl]-2-yl)oxy)dibenzo[d,f][1,3,2]dioxaphosphepin

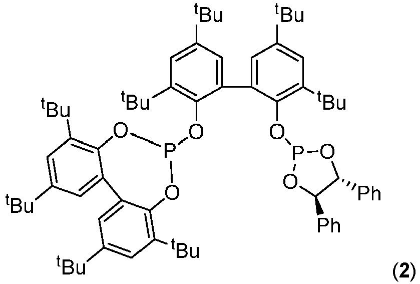

Zu einer Lösung von 3,3',5,5'-Tetra-*tert*.-butyl-2'-((2,4,8,10-tetra-*tert*.-butyldibenzo [d,f][1,3,2]dioxaphosphepin-6-yl)oxy)-[1,1'-biphenyl]-2-ol (0.474 g, 0.558 mmol) in THF (3 ml) wurden bei -20 °C eine Lösung von n-BuLi (0.533 M Lösung in Hexan, 1.10 ml, 0.587 mmol) getropft. Nach 20 min ließ man die Reaktionslösung auf 0 °C erwärmen und versetzte diese anschließend tropfenweise mit einer Lösung von (4*R*,5*R*)-2-chloro-4,5-diphenyl-1,3,2-dioxaphospholane (0.163 g, 0.587 mmol) in THF (3 ml). Nach dem Rühren über Nacht bei Raumtemperatur wurde das Lösemittel unter Vakuum entfernt und der weiße Rückstand mit Toluol (7 ml) versetzt und die ungelösten Bestandteile abfiltriert. Das Filtrat wurde erneut unter Vakuum eingeengt und das Rohprodukt bei 60 °C getrocknet. Zur Reinigung wurde dieser in Acetonitril in der Siedehitze gelöst, nach Abkühlen der ausgefallene Niederschlag abfiltriert und das Produkt als weißer Feststoff isoliert (0.39 g, 0.357 mmol, 64% Ausbeute).

Elementaranalyse (berechnet für C₇₀H₉₂O₆P₂ = 1091.448 g/mol): C = 77.22 % (77.03 %); H = 8.57 % (8.50 %); P = 5.50 % (5.68 %).
ESI-TOF HRMS: m/z= 1090.6369
[M⁺+H], berechnet m/z= 1091.6448 (gefunden 1091.6449)
[M⁺+Na], berechnet m/z= 1113.6262 (gefunden 1113.6276)

### Katalyseversuche

Die Hydroformylierung wurde in einem mit Druckkonstanthaltung, Gasflussmessung, Begasungsrührer und Druckpipette ausgestatteten 200 ml-Autoklaven der Fa. Premex Reactor AG, Lengau, Schweiz, durchgeführt. Zur Minimierung eines Einflusses von Feuchtigkeit und Sauerstoff wurde das als Solvens benutzte Toluol in einem Pure Solv. MD-7 System gereinigt und unter Argon aufbewahrt. Das als Substrat eingesetzten Olefin cis/trans-2-Penten (Aldrich) wurden über Natrium am Rückfluss erhitzt und unter Argon destilliert. Im Autoklav wurde unter Argon-Atmosphäre Lösungen der Katalysatorvorstufe und des Liganden, jeweils in Toluol, gemischt. Als Katalysatorvorstufe kam Rh(acac)(COD) (Umicore, acac = Acetylacetonat-Anion; COD = 1,5-Cyclooctadien) zum Einsatz. Der Autoklav wurde bei 12 bar für den Enddruck von 20 bar unter Rühren (1500 U/min) aufgeheizt. Nach Erreichen der Reaktionstemperatur wurde das Olefin mit einem in der Druckpipette eingestellten Überdruck in den Autoklaven gepresst. Die Reaktion wurde bei konstantem Druck (20 bar) (Nachdruckregler der Fa. Bronkhorst, NL) über 4h geführt. Der Autoklav wurde nach Ablauf der Reaktionszeit auf Zimmertemperatur abgekühlt, unter Rühren entspannt und mit Argon gespült. 1 ml der Reaktionsmischungen wurden unmittelbar nach Abschalten des Rührers entnommen, mit 10 ml Pentan verdünnt und gaschromatographisch analysiert: HP 5890 Series II plus, PONA, 50 m x 0,2 mm x 0,5 µm.

Der Versuch wurde mit den Verbindungen (**1**) und (**2**) durchgeführt.

Die Verbindung (**2**) dient hierbei als Vergleichsligand.

### Ergebnisse der Katalyseversuche

[Rh]: 100 ppm, p: 20 bar, T: 120 °C; t: 4 h; Rh:L = 1:2

**Tabelle: Hydroformylierung von cis/trans-2-Penten**

| Ligand | n-Selektivität [%] |
|---|---|
| (1)* | 83,6 |
| (2) | 74,1 |

| | |
|---|---|
| * erfindungsgemäße Verbindung | |

Die durchgeführten Versuche belegen, dass die gestellte Aufgabe durch eine erfindungsgemäße Verbindung gelöst wird.

## Patentansprüche

1. Verbindung gemäß Formel (I): 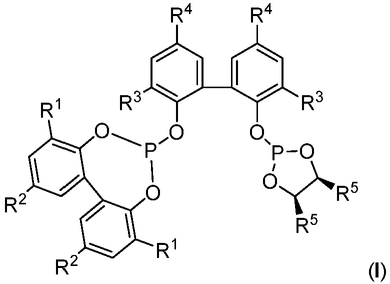
wobei R¹, R², R³, R⁴ ausgewählt sind aus: -(C₁-C₁₂)-Alkyl, -O-(C₁-C₁₂)-Alkyl und
R⁵ ausgewählt ist aus: -(C₁-C₁₂)-Alkyl, -O-(C₁-C₁₂)-Alkyl, -Ph.

2. Verbindung nach Anspruch 1,
wobei R¹ ausgewählt ist aus: -CH₃, -OCH₃, -*^{tert}*Bu.

3. Verbindung nach einem der Ansprüche 1 oder 2,
wobei R¹ für -^{*ter*t}Bu steht.

4. Verbindung nach einem der Ansprüche 1 bis 3,
wobei R² ausgewählt ist aus: -CH₃, -OCH₃, -*^{tert}*Bu.

5. Verbindung nach einem der Ansprüche 1 bis 4,
wobei R² für -*^{tert}*Bu steht.

6. Verbindung nach einem der Ansprüche 1 bis 5,
wobei R³ ausgewählt ist aus: -CH₃, -OCH₃, -*^{tert}*Bu.

7. Verbindung nach einem der Ansprüche 1 bis 6,
wobei R³ für -^{*ter*t}Bu steht.

8. Verbindung nach einem der Ansprüche 1 bis 7,
wobei R⁴ ausgewählt ist aus: -CH₃, -OCH₃, -*^{tert}*Bu.

9. Verbindung nach einem der Ansprüche 1 bis 8,
wobei R⁴ für -^{*ter*t}Bu steht.

10. Verbindung nach einem der Ansprüche 1 bis 9,
wobei R⁵ ausgewählt ist aus: -CH₃, -OCH₃, -*^{tert}*Bu, -Ph.

11. Verbindung nach einem der Ansprüche 1 bis 10,
wobei R⁵ für -Ph steht.

12. Verbindung nach einem der Ansprüche 1 bis 11,
wobei die Verbindung die Struktur (1) aufweist:

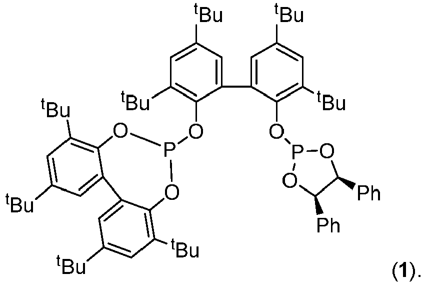

13. Verfahren umfassend die Verfahrensschritte:
a) Vorlegen eines Olefins;
b) Zugabe einer Verbindung nach einem der Ansprüche 1 bis 12;
c) Zugabe einer Rh-Verbindung;
d) Zuführen von H₂ und CO;
e) Erwärmen des Reaktionsgemisches aus a) bis d), wobei das Olefin zu einem Aldehyd umgesetzt wird.

14. Verfahren nach Anspruch 13,
wobei die Rh-Verbindung ausgewählt ist aus: Rh(acac)(CO)₂, [(acac)Rh(COD)] (Umicore, acac = Acetylacetonat-Anion; COD = 1,5-Cyclooctadien), Rh₄CO₁₂.

15. Verfahren nach einem der Ansprüche 13 oder 14,
wobei die Rh-Verbindung Rh(acac)(COD) ist.

## Claims

1. Compound of formula (I): 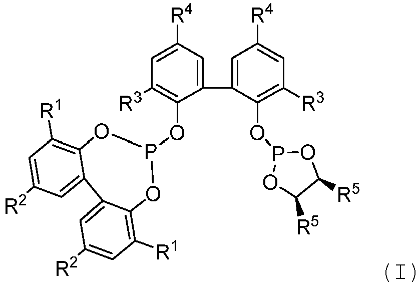
where R¹, R², R³, R⁴ are selected from: -(C₁-C₁₂) -alkyl, - O-(C₁-C₁₂)-alkyl, and
R⁵ is selected from: -(C₁-C₁₂)-alkyl, -O-(C₁-C₁₂)-alkyl, - Ph.

2. Compound according to Claim 1,
wherein R¹ is selected from: -CH₃, -OCH₃, -*^{tert}*Bu.

3. Compound according to either of Claims 1 and 2, wherein R¹ is -*^{tert}*Bu.

4. Compound according to any of Claims 1 to 3, wherein R² is selected from: -CH₃, -OCH₃, -*^{tert}*Bu.

5. Compound according to any of Claims 1 to 4, wherein R² is -*^{tert}*Bu.

6. Compound according to any of Claims 1 to 5, wherein R³ is selected from: -CH₃, -OCH₃, -*^{tert}*Bu.

7. Compound according to any of Claims 1 to 6, wherein R³ is -*^{tert}*Bu.

8. Compound according to any of Claims 1 to 7, wherein R⁴ is selected from: -CH₃, -OCH₃, -*^{tert}*Bu.

9. Compound according to any of Claims 1 to 8, wherein R⁴ is -*^{tert}*Bu.

10. Compound according to any of Claims 1 to 9, wherein R⁵ is selected from: -CH₃, -OCH₃, -*^{tert}*Bu, -Ph.

11. Compound according to any of Claims 1 to 10, wherein R⁵ is -Ph.

12. Compound according to any of Claims 1 to 11, wherein the compound has the structure (1):

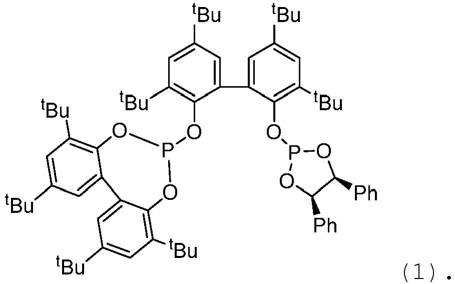

13. Process comprising the process steps of:
a) initially charging an olefin;
b) adding a compound according to any of Claims 1 to 12;
c) adding a Rh compound;
d) feeding in H₂ and CO;
e) heating the reaction mixture from a) to d), to convert the olefin to an aldehyde.

14. Process according to Claim 13,
wherein the Rh compound is selected from: Rh(acac) (CO)₂, [(acac)Rh(COD)] (Umicore, acac = acetylacetonate anion; COD = 1,5-cyclooctadiene), Rh₄CO₁₂.

15. Process according to either of Claims 13 and 14, wherein the Rh compound is Rh(acac) (COD).

## Revendications

1. Composé selon la formule (I) : 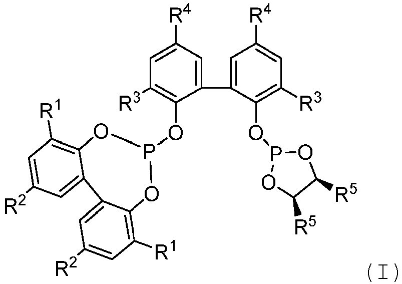
dans laquelle R¹, R², R³, R⁴ sont choisis parmi : - alkyle (C₁-C₁₂), -O-alkyle(C₁-C₁₂) et
R⁵ est choisi parmi : -alkyle (C₁-C₁₂), -O-alkyle (C₁-C₁₂), -Ph.

2. Composé selon la revendication 1,
dans lequel R¹ est choisi parmi : -CH₃, -OCH₃, -tert-Bu.

3. Composé selon l'une quelconque des revendications 1 et 2,
dans lequel R¹ représente -tert-Bu.

4. Composé selon l'une quelconque des revendications 1 à 3,
dans lequel R² est choisi parmi : -CH₃, -OCH₃, -tert-Bu.

5. Composé selon l'une quelconque des revendications 1 à 4,
dans lequel R² représente -tert-Bu.

6. Composé selon l'une quelconque des revendications 1 à 5,
dans lequel R³ est choisi parmi : -CH₃, -OCH₃, -tert-Bu.

7. Composé selon l'une quelconque des revendications 1 à 6,
dans lequel R³ représente -tert-Bu.

8. Composé selon l'une quelconque des revendications 1 à 7,
dans lequel R⁴ est choisi parmi : -CH₃, -OCH₃, -tert-Bu.

9. Composé selon l'une quelconque des revendications 1 à 8,
dans lequel R⁴ représente -tert-Bu.

10. Composé selon l'une quelconque des revendications 1 à 9,
dans lequel R⁵ est choisi parmi : -CH₃, -OCH₃, -tert-Bu, -Ph.

11. Composé selon l'une quelconque des revendications 1 à 10,
dans lequel R⁵ représente -Ph.

12. Composé selon l'une quelconque des revendications 1 à 11,
le composé présentant la structure (1) :

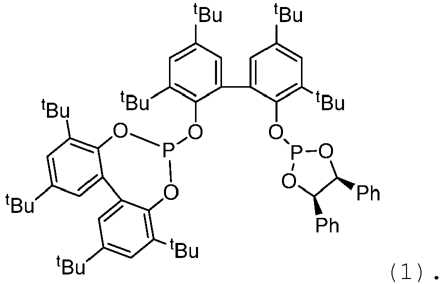

13. Procédé comprenant les étapes de processus :
A) disposition préalable d'une oléfine ;
B) addition d'un composé selon l'une quelconque des revendications 1 à 12 ;
C) addition d'un composé de Rh ;
D) introduction de H₂ et CO ;
F) chauffage du mélange réactionnel provenant de a) à d), l'oléfine étant convertie en un aldéhyde.

14. Procédé selon la revendication 13,
le composé de Rh étant choisi parmi : Rh(acac) (CO)₂, [(acac)Rh(COD)](Umicore, acac = anion acétylacétonate ; COD = 1,5-cyclooctadiène), Rh₄CO₁₂.

15. Procédé selon l'une quelconque des revendications 13 et 14,
dans lequel le composé de Rh est Rh(acac) (COD).
